Europäisches Patentamt

European Patent Office

Office européen des brevets

(11) Publication number: **0 275 398 B1**

# EUROPEAN PATENT SPECIFICATION

(45) Date of publication of patent specification: **05.08.92**  (51) Int. Cl.⁵: **G01N 33/84**, C12Q 1/40, C12Q 1/34

(21) Application number: **87116956.1**

(22) Date of filing: **17.11.87**

The title of the invention has been amended.

(54) **Reagent for the determination of chloride ions.**

(30) Priority: **17.11.86 JP 271775/86**

(43) Date of publication of application:
**27.07.88 Bulletin 88/30**

(45) Publication of the grant of the patent:
**05.08.92 Bulletin 92/32**

(84) Designated Contracting States:
**DE FR GB**

(56) References cited:
**DE-A- 3 323 245**
**DE-A- 3 614 470**
**US-A- 3 597 322**

(73) Proprietor: **Kanto Kagaku Kabushiki Kaisha**
**7, Nihonbashihoncho 3-chome Chuo-ku**
**Tokyo(JP)**

(72) Inventor: **Ono, Toshihiro**
**13-1, Mita 1-chome Tama-ku**
**Kawasaki-shi Kanagawa-ken(JP)**
Inventor: **Taniguchi, Junichi**
**20-17, Watarida 2-chome Kawasaki-ku**
**Kawasaki-shi Kanagawa-ken(JP)**

(74) Representative: **Vossius & Partner**
**Siebertstrasse 4 P.O. Box 86 07 67**
**W-8000 München 86(DE)**

EP 0 275 398 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

A reagent for the determination of chloride ion which comprises a compound capable of forming a chelate with calcium ion, deactivated α-amylase, a calcium chelate compound and an α-amylase activity-measuring substance. This reagent utilizes an enzymatic assay of chloride ion based on the determination of α-amylase activity which depends on the quantity of chloride ion. The reagent of this invention can be used in any type of automatic analytical systems for precise determination of chloride ion contained in serum and the like body fluid and so is very useful in the diagnostic field for checking the metabolic function of electrolytes in living body.

The present invention relates to a reagent for the determination of chloride ion, which is effectively utilizable in the fields of analytical chemistry and diagnosis. More particularly, the present invention relates to a reagent for the determination of chloride ion, which utilizes the nature of α-amylase convertible from its deactivated form into its active form in the presence of chloride ion.

In the field of chemistry, a great number of reports have been submitted hitherto over a century with respect to analysis of chloride, including a primitive routine method for determination of chloride ion with a soluble silver salt and the use of toluidine for colorimetry of free chlorine. Recently, determination of chloride ion is utilized in a wide variety of fields, including precise chemical analysis and diagnosis in medical fields. Especially, determination of chloride ion in serum is clinically important in grasping the metabolic function of electrolytes in living body. Methods for the determination of chloride ion in samples now widely spread for clinical laboratories utilize either of (A) the electric methods, such as coulometric titration [velapoldi RA, Paule RC, Schaffer Re et al. "A reference method for the determination of chloride in serum" Nat. Bur. Stand. (U.S.) Special Publication 260-67 (1979)] and ion selective electrode method [Oesch U, Ammann D, Simon W. "Ion-selective membrane electrodes for clinical use" Clin. Chem. 1986 (32) 1448-1459], wherein the concentration of chloride ion is measured as changes in electric signals and (B) the chemical methods, such as colorimetry [Schales O, Schales SS. "A simple and accurate method for the determination of chloride in biological fluids" J. Biol. Chem. 1941, 140, 879; Sendroy J. "Note on the Photoelectric microdetermination of chloride in biological fluids" J. Biol. Chem. 1942 142, 171-173; Zall DM, Fisher D, Garner MO. "Photometric determination of chlorides in water" Anal. Chem. ]956, (28) 1665-1668], wherein the concentration of chloride ion is measured as charges in color density. Among these conventional methods for determination of chloride ion, the coulometric titration method usually employed in a manual or semi-automatic method is considered to be most reliable but requires time for titration. This method can hardly be set in an automatic analytical system. The ion selective electrode method involves some maintenance problem because of its silver electrodes and also fails to meet the requisites for being used in automatic analytical system.

As a simple method for the determination of chloride ion, the colorimetry is recommended since the measuring operation can be carried out with a simple device. The typical colorimetric methods quoted above are based on the principle that thiocyanate ion formed by the reaction between mercuric thiocyanate (rhodanide) and chloride ion forms a complex with ferric ion and gives a characteristic red orange color effective for colorimetry. The red orange color becomes deeper as the concentration of chloride ion becomes higher. However, particular attention has to be paid in this colorimetric method for handling chemicals for analysis because the chemicals contain harmful mercuric ion and thiocyanate ion. Thus, waste solution or residue from the apparatus used in this method causes serious environmental pollution. Special devices and treatments are therefore necessary for the treatment of such waste solution and residue, thus making this colorimetric method practically less attractive.

On the other hand, it is desirable that the method for the determination of chloride ion can be carried out with a spectrophotometer widely employed in laboratories. Recently, it is highly desired to assemble the device for the determination of chloride ion in an automatic analytical system.

Under the above mentioned circumstances, there is a great demand for developing a new type method or device for the determination of chloride ion in a simple and precise manner using a conventional measuring device and applicable to an automatic analytical system without any problem of environmental pollution as seen in the prior art colorimetric methods.

Accordingly, it is an object of the present invention to provide a new type reagent for the indirect determination of chloride ion by utilizing enzymatic assay of a α-amylase.

It is another object of the present invention to provide a reagent for the determination of chloride ion in a simple manner without paying any attention to environmental pollution for the use of the reagent.

It is still another object of the present invention to provide a reagent for the determination of chloride ion which is utilizable in any type of spectrophotometers for general use and automatic analytical system for clinical use.

Other and further objects, features and advantages of the present invention will be apparent more fully from the following description.

As a result of extensive researches made by the present inventors to develop a new type reagent for the determination of chloride ion, it has now been found surprisingly that deactivated $\alpha$-amylase (referred to hereinafter simply as the deactivated AMY) is converted into the active form combined with calcium in the presence of chloride ion whereby the quantity of the active form of $\alpha$-amylase is exactly proportional to the concentration of chloride ion at a certain concentration and that the concentration of chloride ion can be measured by the enzymatic determination of the active $\alpha$-amylase. The present invention has been accomplished on the basis of the above finding.

In accordance with the present invention, there is provided a reagent for the determination of chloride ion which comprises a compound capable of forming a chelate with calcium ion, deactivated $\alpha$-amylase, a calcium chelate compound and an $\alpha$-amylase activity-measuring substance.

The present invention has various features as compared with the conventional colorimetry or coulometric titration methods; the indirect determination of chloride ion according to the reagent of this invention can be regarded as an enzymatic assay of chloride ion based on the determination of $\alpha$-amylase activity which depends on the quantity of chloride ion.

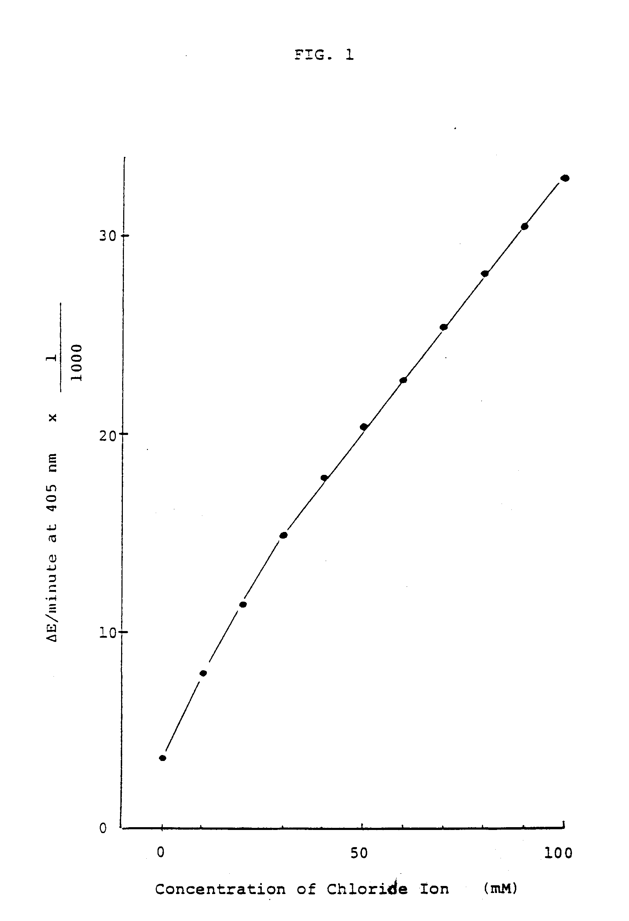

Fig. 1 is a graph showing a calibration curve prepared according to Example 1-(2) wherein the abscissa stands for the concentration of chloride ion in terms of m-Mol (mM) and the ordinate for increase in absorbance ( E/min) at 405 nm.

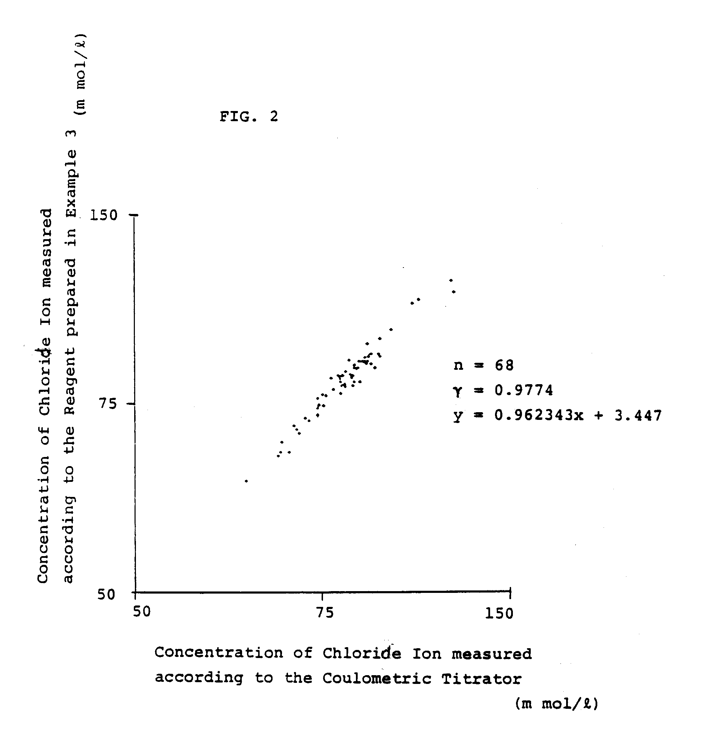

Fig. 2 is a graph showing the correlation in the measurement of the concentration of chloride ion between the present invention and the coulometric titration method, wherein the abscissa stands for the value measured according to the coulometric titrator and the ordinate for the value measured according to Hitachi 705 automatic analytical system using the reagent prepared in Example 3 and 68 human serum samples.

It is known that $\alpha$-amylase (referred to hereinafter simply as AMY) exists usually in the state coupled with calcium ion (active form). (See, for example, "Amylase" compiled by Michinori Nakamura and published on January 1986 by Gakkai Shuppan Center, Japan.) This $\alpha$-amylase in active form will be referred to hereinafter as the active AMY. However, this active AMY is converted in the majority of cases into the deactivated form while releasing calcium ion in the presence of a compound capable of forming a chelate with calcium ion (referred to hereinafter simply as the calcium chelator) such as ethylenediaminetetraacetic acid (EDTA) having a high concentration. It is also known that when this deactivated AMY is reacted with chloride ion at a high concentration (e.g. 10 mM), the deactivated AMY is again coupled with calcium ion to form the active AMY (A. Levitzki and M. L. Steer, Eur. J. Biochem. 41, p.171, 1974).

According to the present inventor's discovery that the quantity of the active AMY is exactly proportional to the concentration of chloride ion within a certain range, the determination of chloride ion can be attained in the present invention indirectly by an enzymatic assay of the active AMY in the following new manner: In the first step, a sample containing chloride ion to be measured is mixed with the deactivated AMY whereby the deactivated AMY is converted into the active AMY only in a quantity corresponding to the concentration of chloride ion existing in the sample. In the second step, the quantity of the active AMY is measured by an AMY activity-measuring substance. The determination of chloride ion can thus be made indirectly by calculating the quantity of chloride ion in the sample from the quantity of the measured active AMY in a proportional relation.

The reagent of the present invention contains, in addition to the deactivated AMY, the calcium chelator, the calcium chelate compound and the AMY activity-measuring substance.

Illustrative of the calcium chelator are, for example, ethylenediaminetetraacetic acid (EDTA), trans-1,2-cyclohexanediamine-N,N,N′,N′-tetraacetic acid, glycoletherdiaminetetraacetic acid, iminotetraacetic acid, diaminopropanetetraacetic acid. The use of EDTA is preferable.

The calcium chelate compound is in principle a chelate of the above calcium chelator with calcium ion. Thus, examples of the calcium chelate compound include calcium ethylenediaminetetraacetate (Ca-EDTA), calcium trans-1,2-cyclohexanediamine-N,N,N′,N′-tetraacetate, calcium iminotetraacetate and calcium diaminopropanetetraacetate. The use of Ca-EDTA is preferable.

Any of the known $\alpha$-amylase activity-measuring substances can be used as the AMY activity-measuring substance used in the present invention. A substance comprised of (a) a substrate of AMY coupling a colorant such as 4-nitrophenol or 2-chloro-4-nitrophenol to an oligosaccharide and (b) a coupled enzyme of $\alpha$-glucosidase and a combination of $\alpha$- and $\beta$-glucosidases, which can hydrolyze an intermediate product formed by hydrolysis of the substrate by the action of $\alpha$-amylase, such as 4-nitrophenyl-$\alpha$-D-maltose, 4-nitrophenyl-$\alpha$-D-maltotriose, 2-chloro-4-nitrophenyl-$\beta$-D-maltose and 2-chloro-4-nitrophenyl-$\beta$-D-maltotriose,

3

to the final product, i.e. 4-nitrophenol or 2-chloro-4-nitrophenol, is desirably used in the present invention. Illustrative of the α-amylase activity-measuring substance are, for example, a substance comprised of 4-nitrophenyl-α-D-maltopentaoside as substrate and α-glucosidase as coupled enzyme, a substance comprised of 2-chloro-4-nitrophenyl-β-D-maltopentaoside as substrate and α-glucosidase and β-glucosidase as coupled enzyme, a substance comprised of 4-nitrophenyl-α-D-maltoheptaoside as substrate and α-glucosidase as coupled enzyme, and a substance comprised of 2-chloro-4-nitrophenyl-β-D-maltoheptaoside as substrate and α-glucosidase and β-glucosidase as coupled enzyme.

The reason why the calcium chelator has to be present in the reagent of this invention, is to stabilize the deactivated AMY used as the essential component in the reagent. As the calcium chelate compound is contained in the reagent of this invention, a very small portion of the deactivated AMY is supplied with calcium ion and is converted in the reagent into the active AMY even in the absence of chloride ion. Practically, this phenomenon is reflected as a blank reagent (blank reaction) in the data obtained and apparently causes inaccuracy of the measurement. In the absence of chloride ion, the existence of the calcium chelator effectively inhibits the reverse reaction of the deactivated AMY to the activated AMY and thus prevents the above mentioned undesirable phenomenon.

No special limitation exists in the proportion of the components used in the reagent. The quantity of the deactivated AMY is preferably larger than the corresponding quantity of the chloride ion to be measured.

As the measurement of the AMY activity is made by an enzymatic reaction, the pH value of the reagent is preferably adjusted to an optimum value for the enzymatic reaction by using a butter substance. Any of the conventional buffer substances can be used for this purpose. Examples of such buffer substance include phosphate buffer, tris-HCl and citrate buffer.

Using the reagent of this invention, the determination of chloride ion is carried out in the following manner: The reagent of the invention is mixed with a series of test samples containing given gradient concentrations of chloride ion. The quantity of the active AMY in proportion to the concentration of chloride ion is measured for each sample as AMY activity. A calibration curve is then prepared in a graph on the basis of the relation between the AMY activity and the concentration of chloride ion. Next, the reagent of this invention is used for a sample containing an unknown concentration of chloride ion. Using the calibration curve, the concentration of chloride ion can easily be obtained.

The concentration of chloride ion within a range of about 10 -3000 mM in a sample can be measured with the reagent of this invention. This range fully covers the concentration of chloride ion in serum usually within a range of 70 - 130 mM.

According to this invention, the determination of chloride ion can be carried out using any type of spectrophotometers widely spread in chemical and clinical laboratories. The reagent of this invention can be utilized in an automatic analytical system for serum recently installed in many clinical laboratories. In addition, the reagent of this invention is free from any harmful substance such as mercuric ion or thiocyanate ion. Thus, no particular attention is necessary for the treatment of waste liquid from the assay unlike the conventional colorimetry.

In addition to ordinary chemical samples containing chlorides various kinds of biological and clinical samples such as serum, plasma, urine and the like can be used for the determination of chloride ion as a sign of metabolic function of electrolytes. Thus, the reagent of this invention finds a wide application to various fields of industry where determination of chloride ion is necessary.

The present invention will now be illustrated in more detail by way of Examples wherein Example 1 illustrates the method for preparing a calibration curve, Example 2 illustrates the determination of chloride ion in human serum with a spectrophotometer and Example 3 illustrates application of the reagent of this invention to an automatic analytical system for determination of chloride ion in human sera.

Example 1 (Preparation of the reagent and a calibration curve)

In this example, α-glucosidase, β-glucosidase and 2-chloro-4-nitrophenyl-β-D-maltoheptaoside were used as the α-amylase measuring reagent in such manner that the α-glucosidase and β-glucosidase were used as components of the ingredient (B) in consideration of their stability with the lapse of time, and that the 2-chloro-4-nitrophenyl-β-D-maltoheptaoside was used as a component of the ingredient (C).

(1) Preparation of the reagent:

Each ingredient of the reagent was prepared in the following manner:

(A) Preparation of the deactivated AMY:

4

α-Amylase extracted from pig pancreas was dialyzed for 24 hours in a 0.1 M phosphate buffer solution (pH 7.0) containing 0.01 M EDTA to prepare a deactivated AMY.

(B) Preparation of a solution containing a chelate compound containing calcium ion and a calcium chelator:

To 100 ml of a 0.1 M phosphate buffer solution (pH 6.2) were added 15 mg of calcium ethylenediaminetetraacetate as the calcium chelate compound and 1.7 g of disodium ethylenediaminetetraacetate as the calcium chelator. After adding to the buffer solution a 5% aqueous solution of sodium hydroxide to adjust the pH value of the buffer solution to 6.9, 11 KU of α-glucosidase and 300 U of β-glucosidase (enzymes) were added to the solution.

To 100 ml of the resultant solution was added 500 U of the deactivated AMY prepared in the foregoing step (A) to prepare Solution R1.

(C) Preparation of the α-amylase activity measuring reagent:

1.0 g of 2-chloro-4-nitrophenyl-β-D-maltoheptaoside (substrate) was dissolved in 100 ml of distilled water to prepare Solution R2.

(2) Preparation of a calibration curve:

First of all, a series of edible salt solutions of gradient concentration were prepared as samples each containing a given concentration of chloride ion. Namely, aqueous solutions of sodium chloride each containing 0, 10, 20, 30, 40, 50, 60, 70, 80, 90 and 100 mM of sodium chloride were prepared as samples. 0.1 Milliliter of each sample was added to 2,0 ml of the Solution R1 and then 0.5 ml of the Solution R2 was added to each sample. Using a spectrophotometer (Model 3200, Hitachi), increase in absorbance of the sample with the lapse of time (ΔE/min) at a wave length of 405 nm were measured at 37°C.

Using an ordinate to express ΔE/min and an abscissa to express the concentration of chloride ion in a graph, the measured values are plotted in the graph to prepare a calibration curve, which is shown in Fig. 1. In this calibration curve, the concentration of chloride ion is exactly proportional to the measured ΔE/min value at a chloride ion concentration of at least 30 mM. Thus, it is possible to determine chloride ion exactly with this calibration curve which is rather linear at a chloride ion concentration above 30 mM.

Example 2 (Determination of chloride ion in human sera)

Using a human serum as a sample, increase in absorbance at 405 nm with the lapse of time (ΔE/min) of the sample was measured according to the method (2) described in Example 1 wherein a combination of Solutions R1 and R2 is used as the reagent for measuring the active α-AMY. The concentration of chloride ion in the serum sample (S-1) in terms of mM was then calculated according to the calibration curve prepared in Example 1(2). In the same manner using the same serum sample, the concentration of chloride ion contained therein was measured repeatedly for 19 times under the same conditions, and the 20 values (S-1 to S-20) measured where statistically treated to calculate their mean value ($\bar{x}$), standard deviation (SD) and coefficient value (CV). Table 1 below shows the calculated values for these samples.

## Table 1

## Tests for coefficient value (CV)

| Sample | Cl concentration | Sample | Cl concentration |
|--------|------------------|--------|------------------|
| S-1 | 100 mM | S-11 | 100 mM |
| S-2 | 100 mM | S-12 | 101 mM |
| S-3 | 99 mM | S-13 | 101 mM |
| S-4 | 100 mM | S-14 | 101 mM |
| S-5 | 98 mM | S-15 | 103 mM |
| S-6 | 100 mM | S-16 | 101 mM |
| S-7 | 99 mM | S-17 | 102 mM |
| S-8 | 100 mM | S-18 | 100 mM |
| S-9 | 99 mM | S-19 | 100 mM |
| S-10 | 102 mM | S-20 | 101 mM |

$\bar{x}$: 100.35 mM; SD: 1.18 mM; CV: 1.17%

Example 3 (A reagent for determination of chloride ion for use in an automatic analytical system)

This reagent consists of three ingredients which can be prepared in the following manner:

(A) Lyophilized Ingredient 1 (Powdery Ingredient 1):

In 50 ml of purified water was dissolved 77 mg of glutathion as a stabilizer for the deactivated AMY. After adjusting the pH value of the solution to 7.0 with a 5(w/v) % aqueous solution of sodium hydroxide, 11 KU of $\alpha$-glucosidase, 300 U of $\beta$-glucosidase and 2000 U of the deactivated AMY prepared in accordance with the method described in Example 1-(1)-(A), were added to the solution. The mixture was the subjected to lyophilization to prepared Lyophilized (Powdery) Ingredient 1.

(B) Lyophilized Ingredient 2 (Powdery Ingredient 2):

In 10 ml of purified water were dissolved 0.25 g of 2-chloro-4-nitrophenyl-$\beta$-D-maltoheptaoside and 1.25 g of maltopentose as a sensibility-adjusting agent. The liquid mixture was then subjected to lyophilization to prepare Lyophilized (Powdery) Ingredient 2.

(C) Buffer Solution (Liquid Ingredient 3):

In about 900 ml of purified water were dissolved 17.4 g of potassium dihydrogen phosphate, 13.6 g of tetrasodium ethylenediaminetetraacetate tetrahydrate, 0.3 g of calcium ethylenediaminetetraacetate dihydrate and 5 g of thiourea. After adjusting the pH value of the solution to 7.0 with orthophosphoric acid, purified water was added to the solution to make the whole volume to 1 liter.

On actual use of this reagent, the lyophilized Reagent 1 is dissolved in 100 ml of the Butter Solution of

6

prepare Reagent 1, while the Lyophilized Ingredient 2 is dissolved in 50 ml of the Buffer Solution to prepare Reagent 2. A combination of the above mentioned Reagents 1 and 2 is set in any type of the automatic analytical system,for example, Hitachi 705 analytical system. In this case, the final concentration of the individual components used in an automatic analytical system are as shown in Table 2 with the proviso that the Reagent 1 and the Reagent 2 are used in a volume ratio of 4:1.

Using the reagent for determination of chloride ion prepared in Example 3 for Hitachi 705 automatic analytical system, the sensitivity and the correlation with the coulometric titrator were examined. As a result of the test, the variation speed of absorbance was 0.160/min for chloride ion at a concentration of 100 mM/liter. As shown in Fig. 2, a great correlation is observed, indicating ($\gamma$) = 0.9774 and a regression line of y = 0.962343x + 3.447.


### Table 2

#### The final concentration of the individual components in case of using the reagent prepared in Example 3 for the automatic analytical system

| | |
|---|---|
| α-glucosidase | 88 KU/liter |
| β-glucosidase | 2.4 KU/liter |
| deactivated AMY | 16 KU/liter |
| ethylenediaminetetraacetic acid | 30 mM |
| calcium ethylenediaminetetraacetate | 0.75 mM |
| thiourea | 0.5 (w/v)% |
| glutathion | 2.0 mM |
| 2-chloro-4-nitrophenyl-β-D-maltoheptaoside | 0.75 mM |
| maltopentose | 0.5 (w/v)% |
| a phosphate buffer solution (pH 7.0) | 0.15 M. |

On the preparation of a reagent for an automatic analytical system, it is desirable to stabilize the ingredients of the reagent and adapt the sensitivity of the reagent to the system. In this Example, the deactivated AMY, α-glucosidase and β-glucosidase were lyophilized together with glutathion as an enzyme stabilizer to prepare Powdery Ingredient 1 wherein the stability of the components is maintained. Further, maltopentose was added together with 2-chloro-4-nitrophenyl-β-D-maltoheptoside to the lyophilized Powdery Ingredient 2 for the purpose of adjusting sensitivity.

In this Example, 68 samples of human sera were used to check the correlation.

## Claims

1. A reagent for the determination of chloride ions which comprises a compound capable of forming a chelate with calcium ions, deactivated α-amylase, a calcium chelate compound and an α-amylase activity-measuring substance.

2. A reagent according to claim 1, wherein the compound capable of forming a chelate with calcium ions is ethylenediaminetetraacetic acid, trans-1-2-cyclohexanediamine-N,N,N′,N′-tetraacetic acid, glycolether-

diaminetetraacetic acid, iminotetraacetic acid or diaminopropanetetraacetic acid.

**3.** A reagent according to claim 1, wherein the α-amylase activity-measuring substance is a substance comprised of 4-nitrophenyl-α-D-maltopentoside and α-glucosidase, a substance comprised of 2-chloro-4-nitrophenyl-β-D-maltopentoside and α-glucosidase and β-glucosidase, substance comprised of 4-nitrophenyl-α-D-maltoheptoside and α-glucosidase or a substance comprised of 2-chloro-4-nitrophenyl-β-D-maltoheptoside and α-glucosidase and β-glucosidase.

**4.** Use of the reagent according to claim 1 for the determination of chloride ions contained in a body fluid for diagnostic purpose.

**Revendications**

**1.** Réactif pour la détermination des ions chlorure, qui comprend un composé capable de former un chélate avec les ions calcium, une amylase α désactivée, un composé chélate de calcium et une substance pour mesurer l'activité de l'amylase α .

**2.** Réactif selon la revendication 1, dans lequel le composé capable de former un chélate avec les ions calcium est l'acide éthylènediaminetétraacétique, l'acide trans-1,2-cyclohexane diamine-N,N,N',N'-tétraacétique, l'acide glycoletherdiaminetétraacétique, l'acide iminotétraacétique ou l'acide diaminopropanetétraacétique.

**3.** Réactif selon la revendication 1, dans lequel la substance pour mesurer l'activité de l'amylase α est une substance constituée de 4-nitrophényl-α-D-maltopentoside et de glucosidase α, une substance constituée de 2-chloro-4-nitrophényl-β-D-maltopentoside et de glucosidase α et de glucosidase β , une substance constituée de 4-nitrophényl-α-D-maltoheptoside et de glucosidase α ou une substance constituée de 2-chloro-4-nitrophényl-β-D-maltoheptoside et de glucosidase α et de glucosidase β .

**4.** Utilisation du réactif selon la revendication 1, pour la détermination des ions chlorure contenus dans un fluide corporel à des fins de diagnostic.

**Patentansprüche**

**1.** Reagenz für die Bestimmung von Chloridionen, enthaltend eine zur Chelatbildung mit Calciumionen fähige Verbindung, desaktivierte α-Amylase, eine Calciumchelatverbindung und ein Mittel zur Messung der α-Amylase-Aktivität.

**2.** Reagenz nach Anspruch 1, wobei die zur Chelatbildung mit Calciumionen fähige Verbindung Ethylendiamintetraessigsäure, trans-1,2-Cyclohexandiamin-N,N,N',N'-tetraessigsäure, Glycoletherdiamintetraessigsäure, Iminotetraessigsäure oder Diaminopropantetraessigsäure ist.

**3.** Reagenz nach Anspruch 1, wobei das Mittel zur Messung der α-Amylase-Aktivität 4-Nitrophenyl-α-D-maltopentosid und α-Glucosidase oder 2-Chlor-4-nitrophenyl-β-D-maltopentosid, α-Glucosidase und β-Glucosidase oder 4-Nitrophenyl-α-D-maltoheptosid und α-Glucosidase oder 2-Chlor-4-nitrophenyl-β-D-maltoheptosid, α-Glucosidase und β-Glucosidase enthält.

**4.** Verwendung des Reagenzes nach Anspruch 1 zur Bestimmung von Chloridionen in Körperflüssigkeit für diagnostische Zwecke.

FIG. 1

FIG. 2

n = 68

γ = 0.9774

y = 0.962343x + 3.447

Concentration of Chloride Ion measured according to the Reagent prepared in Example 3 (m mol/ℓ)

Concentration of Chloride Ion measured according to the Coulometric Titrator

(m mol/ℓ)

10